# EUROPÄISCHE PATENTSCHRIFT

(11) **EP 2 262 471 B1**
(45) Veröffentlichungstag und Bekanntmachung des Hinweises auf die Patenterteilung: **28.10.2015**
(21) Anmeldenummer: 09716740.7
(22) Anmeldetag: 06.03.2009
(51) Int. Cl.: A61K 36/889, A61Q 5/08, A61K 45/06, A61K 31/215, A61K 31/56, A61K 31/5685, A61K 36/25, A61K 36/32, A61K 8/63, A61K 8/97, A61Q 7/00, A61Q 7/02, A61K 36/185, A61K 36/19, A61K 36/48, A61K 36/31, A61K 36/42, A61K 36/13, A61P 17/14

(54) **ZUSAMMENSETZUNG UND VERWENDUNGEN ZUR BEEINFLUSSUNG DES HAARWACHSTUMS**
COMPOSITION AND USES FOR INFLUENCING HAIR GROWTH
COMPOSITION ET UTILISATIONS DE CELLE-CI POUR AGIR SUR LA CROISSANCE DES POILS

(30) Priorität: 07.03.2008 DE 102008012988
(43) Veröffentlichungstag der Anmeldung: 22.12.2010
(73) Patentinhaber: LUCOLAS-M.D. Ltd., Birmingham B18 6EW (GB)
(72) Erfinder: SCHMIDT, Alfred, 79183 Waldkirch (DE)
(74) Vertreter: Prüfer & Partner GbR European Patent Attorneys
(86) Internationale Anmeldenummer: PCT/EP2009/001644
(87) Internationale Veröffentlichungsnummer: WO 2009/109402

(56) Entgegenhaltungen:
- EP-A1- 0 163 490
- EP-A1- 1 700 617
- WO-A1-96/08231
- WO-A1-99/22728
- WO-A1-2006/066354
- WO-A2-00/56269
- WO-A2-01/12206
- WO-A2-2004/034820
- WO-A2-2007/030341
- DE-A1- 4 330 597
- US-A- 6 017 893
- US-A1- 2004 213 859
- US-A1- 2007 036 742
- "Chemicals and their Biological Activities in: Glycine max (L.) MERR. (Fabaceae) -- Soybean" INTERNET CITATION Seiten 1-38, XP007914281 Gefunden im Internet: URL:http://www.ars-grin.gov/cgi-bin/duke/f armacy2.pl [gefunden am 2010-08-04]
- BRODIE A M: "Overview of recent development of aromatase inhibitors" STN MEDLINE, 1. August 1982 (1982-08-01), XP002104192
- H-J JEONG ET AL: "Inhibition of aromatase activity by flavonoids" ARCHIVES OF PHARMACAL RESEARCH, NATL. FISHERIES UNIVERSITY, PUSAN, KR LNKD- DOI:10.1007/BF02976369, Bd. 22, Nr. 3, 1. Januar 1999 (1999-01-01) , Seiten 309-312, XP002536317 ISSN: 0253-6269
- MARTY E SAWAYA AND VERA H PRICE: "Different Levels of 5a-Reductase Type I and II, Aromatase, and Androgen Receptor in Hair Follicles of Women and Men with Androgenetic Alopecia" JOURNAL OF INVESTIGATIVE DERMATOLOGY,, Bd. 109, Nr. 3, 1. September 1997 (1997-09-01), Seiten 296-300, XP009137216 ISSN: 0022-202X
- OSOSKI A L ET AL: "PHYTOESTROGENS: A REVIEW OF THE PRESENT STATE OF RESEARCH", PHYTOTHERAPY RESEARCH, JOHN WILEY & SONS LTD. CHICHESTER, GB, vol. 17, no. 8, 1 January 2003 (2003-01-01), pages 845-869, XP008039293, ISSN: 0951-418X, DOI: DOI:10.1002/PTR.1364

## Beschreibung

Die vorliegende Erfindung bezieht sich auf eine Zusammensetzung, die zur Beeinflussung des Haarwachstums besonders geeignet ist, sowie damit zusammenhängende Verwendungen.

Über viele Jahrzehnte wurde die Erforschung des hormonabhängigen Haarwuchses von Androgenen dominiert. Der Androgen-Metabolismus und die Androgenen-Rezeptoren sind bis heute Ziele sowohl der systemisch pharmakologischen Beeinflussung des Haarwuchses in der Medizin als auch der lokalen, kosmetischen Versuche den Haarwuchs zu kontrollieren.
Dennoch ist es seit langen bekannt, dass auch die Östrogene einen erheblichen Einfluss auf die Haarfollikel bzgl. Wachstum und Zyklus haben. Und zwar über die Bindung an die lokalen Östrogen-rezeptoren.

So konnte beobachtet werden, dass eine Erhöhung der lokalen Östradiolspiegel über eine lokale Östradiolgabe zu einer Steigerung der Körperbehaarung führte. Eine umgekehrte Beobachtung konnte bei der lokalen Gabe von Östradiol auf die Kopfhaut gemacht werden: hier führte der erhöhte Östradiol Spiegel bzw. die erhöhte lokale Östradiol Aktivität zu Haarausfall. Grundlage der positiven Beeinflussung des Wachstums der Kopfbehaarung ist, dass jedes Haarfollikel einen einzigartigen "Mikrokosmos" darstellt, der die Fähigkeit hat, sich selbst vollständig zu regenerieren. Dieses basiert auf den Interaktionen seiner epithelialen und mesenchymalen Komponenten. Diese Interaktionen beziehen sich auch auf die lokale Entstehung und das Zusammenspiel der Sexualhormone.
Diese Mechanismen spielen natürlich, wenn auch teilweise gegensinnig, eine gleich große Rolle bei der Körperbehaarung. Ebenso spielen diese Faktoren neben ihrer regulartorischen Funktion innerhalb des Haarfollikol- Zyklus eine bedeutende Rolle innerhalb der Haarpigmentierung.
Das Schlüsselkonzept ist also, dass im Prinzip Östrogene und Androgene eine direkte haarwuchsmodulierende Funktion haben und indirekt darüber hinaus über die Veränderung der Expression von wichtigen haarwuchsmodulierenden Faktoren wirken. Stütze dieses Konzepts ist das, dass die beiden essentiellen Enzyme für die Genese der aktiven Sexualhormone, Aromatase für die Umwandlung von Testosteron zu Östradiol und die 5-α-Reduktase (Typ I und Typ II) für die Umwandlung von Testosteron zu Dihydrotestosteron im Bereich der Haarfollikel stark expriniert und aktiv sind. Das gleiche gilt für die Expression der Östrogen-Rezeptoren α und β und der Androgen-Rezeptoren. Dieses bedeutet, dass die Haut nicht nur eine schützende und regulierende Funktion hat, sondern die Haut darüber hinaus ein bedeutendes endokrines Organ ist.

In der Vergangenheit gab es Vorschläge, Aromatase-Inhibitoren als kosmetisches Mittel und zur Beeinflussung des Haarwachstums einzusetzen, wie z.B. in der WO 96/08231 A diskutiert.

Extrakte aus Sägepalmen (Serenoa Repens) sind in einer Reihe von Dokumenten in erster Linie im Zusammenhang mit pharmazeutisch therapeutischen Konzepten erwähnt worden, s. z.B. JP 2007 230888 A in Bezug auf Androgen-unabhängigen Krebs; US 2006 246153 A bezüglich der Benignen Prostata-Hypertrophie (BPH); JP 2007 051129 A als Bestandteil einer Zusammensetzung für einen Antagonisten gegenüber dem Angiotensin II1-Typ-Rezeptor oder als Inhibitor des Angiotensin I konvertierenden Enzyms; WO 03/030887 A zur Behandlung von Sexualstörungen bzw. der erektilen Dysfunktion; US 6,599,540 zur Prävention und/oder Behandlung des Prostatakrebses; US 2002 001633 A zur Behandlung u.a. der BPH und des Prostatakrebses; DE 10 127897 zur Behandlung der Osteoporose oder verwandten Erkrankungen; WO 01/39656 A zur Behandlung von Symptomen des unteren Harntrakts (LUTS) und der BPH; FR 2791255 bezüglich anti-androgener Wirkung kosmetischer und dermopharmazeutischer Zusammensetzungen; JP 2000 256204 A bzgl. einer Zusammensetzung mit gesteigerter Prostatomegalie-inhibierender Wirkung; WO 99/21009 A zur Herstellung geeigneter Sägepalmen-Extrakte; WO 97/03639 für topische kosmetische Hautanwendungen; EP-A-0204877 als dermatologische topische Zusammensetzung zur Rehandlung von Akne; jeweils in Verbindung mit anderen Wirkstoffen sowie Trägerstoffen. Daneben befassen sich andere Schriften mit Haaranwendungen, s. z.B. die JP 2002 322050 A mit einer Zusammensetzung von Sägepalmen-Extrakt mit Cystin (Haar-Rohmaterial), Zitronensäure und Theanin zur Aktivierung der Haarwurzel und zur Förderung des Haarwachstums durch orale Verabreichung, die US 2001 033849 A mit kosmetischen Zusammensetzungen, die Fettsäuren und anti-androgene Sterole aus Sägepalmen-Extrakt und/oder Cucurbita-Samen (cucurbita pepo), die US 6,019,976 mit therapeutischen Formulierungen enthaltend Sägepalmen-Extrakt, Vitamin B6, Vitamin B3, Zinksalz und L-Arginin zur Behandlung der männlichen Glatze durch topische Haarapplikation, die JP 11 092340 A mit einer Formulierung aus Sägepalmen-Extrakt und einer speziellen Menge einer öllöslichen Komponente aus Allium sativum L. zur Förderung des Blutstroms und mit einer nachfolgend erwarteten Verbesserung eines Haar erneuernden Effekts und somit zur Behandlung oder Prävention gegen männlichen Haarausfall, der JP 60 215608 A mit einem Sägepalmen-Extrakt für cine Haar stärkende Präparation, die WO 03/013561 A mit pharmazeutischen und/oder kosmetischen Zusammensetzungen enthaltend als wirksame Bestandteile Extrakte aus Sägepalmen und Vitis vinifera zur Behandlung und Prophylaxe des Haarausfalls, von Schuppen und der Seborrhö, sowie die WO 98/33472 mit Sägepalmen-Extrakten oder deren Bestandteilen zur Prävention und/oder Behandlung androgenen Haarausfalls und/oder Hirsutismus.

Die WO 91/02516 A bezieht sich auf eine Verwendung eines Koleusextrakts zur Hautpigmentierung, wobei ergänzende Substanzen mitverwendet werden können wie Xanthine, Theophilin, Tyrosin, Chinin, hautreizende Mittel, 5α-Reduktase-Inhibitoren und einem Extrakt von Sägepalmen, sagt jedoch in Bezug auf die Schrift EP-A-0 293 837, die sich mit der Beeinflussung von Melanozyten der Haarwurzeln und daher zur Behandlung des Haares beschäftigt, dass es zwischen Melanozyten in Haarfollikeln und Melanozyten in der Haut große Unterschiede, insbesondere bezüglich des Stoffwechsels, gibt.

Wiederum andere Dokumente beschäftigen sich mit Versuchen zur Beeinflussung und insbesondere der Hemmung der 5α-Reduktase.

Die US 7238 375 B1 beschreibt vier Komplexe 1-4, wobei Komplexe 1-3 Haarverlust verhindern und Komplex 4 Haarwachstum fördern soll. Zu Komplex 2 gehört eine Mischung aus Kupferionen, Palmenextrakt (*Serenoa repens*), Pygeumextrakt (*Pygeum africanum*), Brennnesselextrak (*Urtica dioica*), Zink, Vitamin B6 und Linolensäure.
US7105573B2 und US2007/0066661A1 betreffen weitere Vorschläge zur Behandlung von Haarausfall und anderen Erkrankungen, die mit Haarfollikeln zusammenhängen.
Der Artikel von W.CHEN et al., "Cutaneous Androgen Metabolism: Basic Research and Clinical Perspectives", J Invest Dermatol. Vol. 119, No. 5, Nov 2002, S. 992-1007 beschreibt allgemein den Androgenmetabolismus der Haut; und R. HOFFMANN und R.HAPPLE, "Current understanding of androgenetic alopecia. Part I: Etiopathogenesis", Eur J Dermatol. Vol 10, No. 4, Juni 2000, S. 319-327 geben einen Überblick über den Kenntnisstand bei der Androgenen Allopezie (AGA), wobei beide genannten Artikel einen Eindruck vom komplexen Sachverhalt der biochemischen Grundlagen in den für die Haut- und Haarphänomene relevanten anatomischen Bereich vermitteln.
M.E. SAWAYA M. E., "Different Levels of 5α-Reductase Type I and II, Aromatase, and Androgen Receptor in Hair Follicles of Women and Men with Androgenetic Alopecia", J Invest Dermatol. Vol. 109, No. 3, Sep 1997, S. 296-300 bereichten über Unterschiede des Vorkommens des Androgenrezeptors und von Steroidumwandelnden Enzymen (5α-Reduktase, Aromatase) bei Mann und Frau und vermuten, dass dies für das unterschiedliche klinische Bild der AGA bei Mann und Frau verantwortlich sein könnte.
Eine Liste von möglichehn Wirkstoffen, die jeweiligen Pflanzen zugeordnet warden können, kann gefunden werden bei Dr. Duke's Phytochemical and Ethnobotanical Databases, URL:http://www.ars-grin.gov/duke.

Wie oben erläutert betrifft die Beeinflussung des Haarwachstums ein äußerst komplexes System. Dies spiegelt sich in einer entsprechend hohen Komplexität von biochemischen Untersuchungen und Vorschlägen zu deren Beeinflussung wider. Trotz der Vielzahl von Versuchen vermochten bisherige Ansätze jedoch kein geeignetes Gleichgewicht zu entwickeln in Bezug auf eine differenzierte lokale Beeinflussung der maßgeblichen Metaboliten wie Östrogene (insbesondere Östradiol) und Androgene (insbesondere Dihydrotestosteron), je nachdem ob das Haarwachstum oder die Haarentfernung - bzw. Verhinderung des Nachwachsens von Haaren nach Epilation oder Depilation - in Abhängigkeit von der behandelten Hautregion, d.h. einerseits Kopfhaut (Skalp) und andererseits Körper einschließlich Gesicht (insbesondere Bartbereich), und je nachdem ob Frauen oder Männer betroffen sind. Eine zufriedenstellende differenzierte Lösung zur gezielten Beeinflussung sowohl beim Mann als auch bei der Frau wurde bisher nicht gefunden. Insbesondere eine Depigmentierung von Haaren stellt ein nichtvorhersehbares Problem bei der Beeinflussung des Haarwachstums dar.

Aufgabe der vorliegenden Erfindung ist es daher, eine verbesserte Zusammensetzung zur Beeinflussung des Haarwachstums und der Haarpigmentierung bereitzustellen.

Die vorliegende Erfindung stellt daher einen Gegenstand wie in den Ansprüchen definiert bereit.

Erfindungsgemäß wurde überraschend gefunden, dass wenn mindestens ein Aromatase-Inhibitor, der aus der Gruppe von Aromatase-Inhibition aufweisenden Extrakten von Raps ausgewählt ist (Komponente (i)), mit mindestens einem Pflanzenextrakt (Komponente (ii)), der eine oder mehrere aus der Pflanze extrahierte Wirkstoffsubstanz(en) enthält, die aus der aus 5α-Reduktase Typ I-und/oder Typ II-Inhibitoren und Androgenrezeptor-Blockern bestehenden Gruppe ausgewählt ist (sind), wobei der mindestens eine Pflanzenextrakt aus der Sägepalme (Serenoa Serrulata Fruit Extract) ist, kombiniert ist, nicht nur eine gezielt steuerbare Beeinflussung des Haarwachstums erreicht wird, sondern gleichzeitig auch die Haarpigmentierung steuert. So kann entweder eine Haardepigmentierung, so bei der Frau, oder andererseits Schutz gegenüber Haardepigmentierung, so beim Mann, erreicht werden.

Die neue erfindungsgemäße Kombination zeichnet sich durch eine vorteilhafte gezielte Beeinflußbarkeit des Haarwachstums aus, und zwar je nach dem, ob Haarwachstum oder Haarentfernung bzw. Verhinderung eines Neuwachstums von Haaren je nach Körperbereich (Skalp/Kopfhaut oder Körper/Gesicht/Bartbereich) und je nach dem ob Frauen oder Männer betroffen sind. Dabei wurde nämlich überraschend festgestellt, dass die erfindungsgemäße Kombination eine gezielte und differenzierte Beeinflussung der für das Haarwachstum maßgeblichen Metaboliten wie Östradiol und Dihydrolestosteron bezogen auf die lokalen Gegebenheiten zuläßt - d.h. gezielt zur Förderung des Haarwachstums auf der Kopfhaut/Skalp bei Mann oder Frau und zur Haarentfernung bzw. Verhinderung des Neuwachstums nach Epilierung oder Depilierung am Körper (einschließlich Bartbereich) bei Mann oder Frau - verbunden mit der Möglichkeit einer günstigen Beeinflussung der Haarpigmentierung und insbesondere Re-Pigmentierung der Haare und damit eine Rückkehr zur ursprünglichen Haarfarbe.

Für die erfindungsgemäße differenzierte Kombinationswirkung ist es wichtig, dass schnell und effizient eine wirksame Aromatasehemmung herbeigeführt wird, weshalb die Komponente (i) ausgewählt wird aus Aromatase-Inhibition aufweisenden Extrakten von Raps.

Als in einer Zusammensetzung verwendbaren, chemischsynthetischem Aromatase-Inhibitor kann eine mit dieser Funktion an sich bekannter Stoff verwendet werden, vgl. z. B. A.M.H. Brodie in: "J. Steorid Biochem. Molec. Biol.", Vol. 49, No. 4-6, pp. 281-287 (1994), sowie P. E. Goss und K.M.E.H. Gwyn in: "Journal of Clinical Oncology", Vol. 12, No. 11, pp. 2460-2470 (1994), und zur Bestimmung der Aromatase-Inhibition s. beispielsweise A.M.H. Brodie et al. in: "J. Steroid Biochem. Molec. Biol.", Vol. 7, pp. 787-793 (1976), und D.A. Marsh et al. in: "J. Med. Chem.", Vol. 28, pp. 788-795 (1985). Geeignete Aromatase-Inhibitoren können zum Beispiel aus der folgenden Gruppe von Verbindungen ausgewählt werden:
Steroidale Aromatasehemmer:
   4-Hydroxyandrost-4-en-3,17-dion (Formestan und Lentaron),
   6-Methylenandrostra-1,4-dien-3,17-dion (Exemestan),
   10-(2-Propynyl)estr-4-en-3,17-dion (MDL 18962)
   7-alpha substituierte Androstendion-Derivate
   1,4,6-Androstatriene-3,17-dion (ATD)
   10-Oxiran- und 10-Thiirane- substituierte Androgene
   10-Propargylestr-4-en-3,17-dion
   10-propargylestr-4-en-3,17-propionat-10-(2-propynyl)-Derivat
   13-retro-Antiprogestin
   14-alpha-Hydroxy-4-androsten-3,6,17-trion (14-alpha-OHAT)
   16- oder 19-substituierte Androst-4-ene
   19-(Cyclopropylamino)-androst-4-en-3,17-dion
   19-(Ethyldithio)-androst-4-en-3,17-dion
   19-Oxiranyl- and 19-Thiiranyl-Steroide
   19-Thiomethyl- and 19-Azido-androstenedion
   1-Methyl-androsta-1,4-dien-3,17-dione (Atamestan)
   2,7.-Dimethyl-4-hydroxy-4-androsten-3,17-dion
   3-alpha-methoxyandrost-4-en-6,17-dion
   3-beta-hydroxyandrost-4-en-6-on-Derivat
   3-Deoxyandrogen-19-Oxygenierderivate von 3-oxo-17 betacarboxamido-Steroiden
   4-(Phenylthio)-4-androsten-3,17-dion
   4-(Thio-substituiertes)-4-androsten-3,17-dion
   4-Acetoxy-4-androsten-3,17-dion
   4-Aminoandrostenedion
   4-Androsten-3,6,17-trion
   4-Hydroxyandrostenedion (4-OHA)
   4-Methoxy-4-androsten-3,17-dion
   4-Oxygenierte Androst-5-en-17-on und deren 7-oxo-Derivate
   4-Thiosubstituierte Derivate von 1-Androsten-3,17-dion
   4-Thiosubstituierte-4-androsten-3,17-dion-Derivate
   5-alpha-Dihydronorethindron (ein Metabolit von Norethindron)
   5-alpha-reduzierte C19-Steroide
   5-alpha-Androstan-17-on mit oder ohne eine Carbonylfunktion an C-3 und/oder C-6
   6-alpha-7-alpha-Cyclopropanderivate von Androst-4-en
   6-alpha-Fluorotestosteron
   6-beta-Propynyl-substituierte Steroide
   6,7-Aziridinylsteroid und verwandte Verbindungen
   6-Alkylanaloge von delta 1,4,6-Androgenen
   6-Alkyl- und 6-Arylandrost-4-en-3,17-dion
   6-Alkylandrost-4-en-3,17-dion von 7 alpha- and 7-betaarylaliphatisch-substituierten Androst-4-en-3,17-dionen
   6-Alkylandrosta-4,6-dien-3,17-dion und deren 1,4,6-trien-Analoga
   6-Alkyl-substituierte Androgene
   6-Phenylaliphatisch-substituierte C19-Steroide mit 1,4-dien-,
   4,6-dien- oder 1,4,6-trien-Struktur
   6-Bromoandrostenedion
   6-Hydroximinoandrostenedion
   6-Methylenandrosta-1,4-dien-3,17-dion (FCE 24304)
   6-Methylenandrosta-1,4-dien-3,17-dion (FCE 24304)
   6-Phenylaliphatisch-substituierte Androst-4-en-3,17-dione
   6-substituierte Androst-4-en-Analoga
   7-alpha-(4'-Amino)phenylthio-4-androsten-3,17-dion
   7-alpha-substituierte Androsta-1,4-dien-3,17-dione
   7-alpha-substituierte Androstenedione
   7-alpha-(4'-Amino)phenylthio-4-androsten-3,17-dion
   7-alpha-arylaliphatische Androsta-1,4-dien-3,17-dione
   7-alpha-substituierte Androstenedione
   7-substituierte 4,6-Androstadien-3,17-dione
   7-substituierte Steroide
   Androst-4-en-3,6-dionderivative
   Androst-5-ene-7,17-dion 19-nor- und 5-beta-6-beta-epoxy- Derivate
   A-oder B-ring-substituierte Derivative von Androst-4-en-3,6,17-trion
   A-ring verbrückte Steroide
   Bromoacetoxy-4-androsten-3-on
   delta-1,4,6-Androgene
   delta-4,6-Androgene
   epimere 6-Hydroperoxyandrostendione
   Estr-4-en-3,17-dion (MDL 18 962),
   Estr-4-en-3,6,17-trion
   Flavonoide
   RU486

Nicht-steroidale Aromatase-Inhibitoren:
6-[(4-Chlorophenyl)(1H-1,2,4-triazol-1-yl)-methyl]-1-methyl-1H-benzotriazol (Vorazol),
2,2'-[5-(1H-1,2,4-triazol-1-yl methyl)-1,3-phenylen]bis(2-methylproprionitril) (Arimidex),
4-[1-(Cyanophenyl)-1-(1,2,4-triazolyl)methyl]benzonitril (Letrozol),
{4-(5,6,7,8-Tetrahydro-imidazo-[1,5a]-pyridin-5-yl) benzonitril Monohydrochlorid (Fadrozol)
Pyridoglutethimid (Rogletimid)
Aminogluthetimid
1,2-Imidazolylmethylcyclopentanol-Derivate
1-[(Benzofuran-2-yl)phenylmethyl]-triazole and -tetrazole
1-[Benzofuran-2-yl)-phenylmethyl]-imidazole (substituiert)
1-(Benzofuran-2-ylmethyl)imidazole von N,N-disubstituierten-5-aminopyrimidin-Derivaten
1-Imidazolyl(alkyl)-substituierte Di- und Tetrahydrochinoline
1-Pentyl-3-(4-aminophenyl)pyrrolidin-2,5-dion
1-Phenyl-3-azabicyclo[3.1.0]hexan-2,4-dion
1-Phenyl-3-azabicyclo[3.1.0]hexan-2,4-dion und Analoga
3-alkylierte 3-(4-Aminophenyl)piperidin-2,6-dione
3-cycloalkyl-substituierte 3-(4-Aminophenyl)piperidin-2,6-dione
3-Ethyl-3-(4-pyridyl)piperidin-2,6- und 5-Alkylderivate
3-Ethyl-3-(4-pyridyl)piperidin-2,6-dion-Analoga
4-Amino-4H-1,2,4-triazol-Derivate
4-Cyclohexylanilin
Aminoglutethimid
Benzimidazol- und Imidazol-Verbindungen
Delta-1,4-Bisnorcholadiensäure
Delta-1-Testolacton
Imidazolderivative von pyrrolidonischen and piperidonischen Imidazolyl-1,3,5-triazinen
MR 20492 and MR 20494 (zwei Indolizinonderivative)
Pyridyl-substituierte Indanone, Indane und Tetraline
Triazinderivat SEF19
Substituierte Pyridine
Testololacton

Andere Aromatase-Inhibitoren:
8-Bromo-cyclisches Adenosinemonophosphat
FR901537
Hexamethylmelamin-Derivat (SAE9)
Letrozole (CGS 20267)
Mefloquin
MPV-2213ad
N-n-Octanoylnornicotin und andere Nornicotinderivate
Org 33201
R 76713 und R 76713
Sesquiterpenlacton
SH 489
TAN-931
Thyroidhormone
Tobakalkaloidderivate
YM511

Hinsichtlich der Bezeichnungen dieser Substanzen sowie deren Verfügbarkeit siehe beispielsweise "Rote Liste", Editio Cantor Verlag, Aulendorf (DE), 2003.

Im Hinblick auf eine Wirksamkeit zur Aromatase-Hemmung einerseits und einer schonenden Behandlung von Hautregionen für eine bevorzugte lokale und topische Applikation der Zusammensetzung andererseits wird ein Raps-Extrakt (Brassica Campestris; engl.: rapeseed) eingesetzt. Die jeweiligen Raps-Extrakte können zum erfindungsgemäßen Einsatz als Aromatase-Inhibitor-Komponente so erhalten werden, dass jeweils Fraktionen durch geeignete Extraktion und ggf. gezielter Abtrennung und Isolierung jene Bestandteile aus Raps bzw. Rapsöl erhalten werden, die eine Aromatase-Hemmeigenschaft aufweisen. Raps-Extrakte können vorteilhafterweise zusätzlich eine 5α-Reduktase-Hemmeigenschaft hervorbringen, was wie nachfolgend beschrieben eine noch vorteilhaftere Wirkungsweise ermöglicht. Die gezielte Isolierung von Extraktfraktionen mit Aromatase- und/oder 5α-Reduktase-Hemmeigenschaften können durch entsprechende Tests der Fraktionen auf die jeweilige Hemmwirkung nachgewiesen werden und entsprechend gesammelt werden, wobei jeweils bekannte spezifische Hemmungs-Assays verwendet werden können.

Die erfindungsgemäße Kombinationswirkung wird dadurch realisiert, dass ferner mindestens ein Pflanzenextrakt verwendet wird, der einen aus der Pflanze extrahierte Wirkstoffsubstanz enthält, die aus der aus 5α-Reduktase Typ I- und/oder Typ II-Inhibitoren und Androgenrezeptor-Blockern bestehenden Gruppe ausgewählt ist, und wobei der mindestens eine Pflanzenextrakt ein Extrakt aus Sägepalme (Serenoa Serrulata Fruit Extract) ist. Der Einsatz des Pflanzenextrakts weist für den erfindungsgemäßen Zweck deutliche Vorteile im Vergleich zum Einsatz einzelner chemisch-synthetischer 5α-Reduktase-Inhibitoren auf und ist bevorzugt, z.B. weil dann steroidale Phytosterole und/oder Flavonoide als erfindungsgemäß besonders günstige Wirkstoffe erhalten werden, und/oder weil dann regelmäßig ein Gesamtgemisch von strukturell und gegebenenfalls funktionell unterschiedlichen Wirkstoffen erhalten werden. Folglich ist erfindungsgemäß insbesondere ein solcher Pflanzenextrakt bevorzugt, der mehrere aus der Pflanze extrahierte Wirkstoffsubstanzen, d.h. ein Wirkstoffgemisch enthält, die aus der aus 5α-Reduktase Typ I- und/oder Typ II-Inhibitoren und Androgenrezeptor-Blockern bestehenden Gruppe ausgewählt sind.

Pflanzenextrakte, die eine Wirkung zur Hemmung der 5α-Reduktase des Typs I- und/oder des Typs II, vorzugsweise beider Typ I- und Typ II-Formen, besitzen, sind insbesondere Extrakte aus folgenden Pflanzen, jeweils allein oder in Kombination: Sägepalmen-Extrakt (Serenoa repens); Taxus chinensis (Pilg.) Rehd., Canarium pimela Koenig, Heteropanax fragrans (Roxb.) Seem., Andrographis paniculata(Burm.f.) Nees, Acer palmatum, Zosteraceae, Zostera sp., Yacon (eine aus Peru stammenden natürlichen Pflanze, die zur Gattung der Asteraceae gehört, mit botanischem Namen: Polymnioa sonchifolia), Sesam, Stachelbeere (Gattung Phyllanthus der Familío Euphorbiaceace, botan. Name: Phyllanthus Emblica), Striga asiatica (L.) O. Kuntze, Butea monosperma (Lam.) Taub., Alangium chinense (Lour.) Harms, Alternanthera sessilis (L.) R. Br., Procryis wightiana wall ex Wedd., Desmodium triflorum (L.) DC., Stephania japonica Miers., Polypodium vulgare, Quercus-Pflanze (Gattung), Psidium guajava L, Plumbago zeylanicum L., Cyperus rotundus L., Ricinus communis L., Embelia ribes Burm. f., Jangkang, Daun trawas, Cuachalalat (stammt aus der Acapulco-Region im Süden Mexikos), Piper Methysticum (Gattung Piper, Familie Piperaceae), Impatiens balsamina L., Thuja orientalis (Familie der Zypresse), Pflanzen der Gattung Coriandrum wie z.R. Corlandrum sativum L., Cassia auriculata (insbesondere Rinde), Quercus pedunculata (insbesondere Früchte), Rumex cyprius, Sumilax zeylanica, Phyllanthus nuriri, Woodfordia fruticosa, Lagerstroemia speciosa, Cymbopogon nardus, Glycyrrhiza glabra oder Rheum, Belamcanda chinensis DC. (Family Iridaceae), Rosa rugosa Thunb, Saxifraga stolonifera Meerburg, Garcinia mangostana L., Nephelium lappaceum L., Pyrola japonica Klenza, Trichosanthes cucumeroides Maxim, Kadsura japonica Dunal, Cuscuta australis R. Dr., Cuscuta japonica Choisy, Euchresta japonica Benth., Lilium makinoi Koidzumi, balbatimone, Rosaceae wie Pfirsich, Rosa rugasa, Rosa odorata, Rosa odorata, R. coptophyllus, Rosa centifolia, sanguisorba officinalis L. oder Pseudocydonia siensis, Lequminosae, Polygoni Multiflori Radix, Chaenomelis Fructus, Zanthoxylic Fructus, Thujae Orientalis, Landium domesticum Jack var. Duku (Duku) oder Landium domesticum Jack var. Langsat (Langsat) der Meliaceae-Familie, Uncaria gambir, Fenchel, Polygala, Süßholz, Pharbitis, Wegerich, Gewürznelke, Arecanuss, Kolophonium, Stachys betonica, Geranium herb, Pounellae spical, Bupleurum elatum, Artenisiae capillaris Flos, Rosae fructus, Coicis semen, Nepetae herba, Dichroa, Valeriana officinalis. Im Rahmen der vorliegenden Erfindung enthält die Zusammensetzung mindestens ein Pflanzenextrakt wie in Anspruch 1, Punkt (ii), angegeben.

Pflanzenextrakte, die eine Wirkung zum Blocken des Androgenrezeptors (Antiandrogen) haben, sind insbesondere folgende Extrakte, jeweils allein oder in Kombination:
Chromolaena odoratum (L.) K. R., Kokosnußöl, Kubanische Königspalme (Roystonea regia), Pygeum africanum, Serenoa repens, Cucurbita pepo, Albizia lebbeck (L.) Benth (aus Rinde), Roystonea regia (aus Früchten), Ruta graveolens L, Azadirachta indica A. Juss (aus Blättern), Momordica charantia (aus Samen), Ganoderma lucidum, Echinacea purpurea, Belamcanda chinensis, Citrus aurantium, Echinacea purpurea, Silybum marianum (Mariendistel), Crotalaria juncea Linn, Pygeum africanum, Kürbiskernöl, Rosenklee [insbesondere Flavonoid-reiche Bestandteile; Trifoleum pratense] Kiefer (Pinus), Fiche (Picea), Roggen (Blütenpollenextrakt), Soja, Pygeum africanum, Hypoxis rooperi (Wurzel), Brennnessel (Urtica dioica), Cordia multispicata (Triterpenoid-ExLrakL) aus Brasilien, Myricae Cortex (Myrica rubra Sieb. et Zucc., Myricaceae, aus Rinde), Pygeum africanum (Tadenan; Rindenextrakt der Afrikanischen Pflaume), Azadirachta indica, Sophora flavescens, Hibiscus rosa sinesis, Dalbergia cochinchinensis, Fructus Psoraleae, Striga orobanchioides, und Vitex negundo (aus Samen).

Vor allem bevorzugt werden Extrakte von Früchten der Sägepalme (Serenoa Serrulata Fruit Extract) (speziell der Ethanolextrakt), von Kürbiskernen, 3rennessel, Taxus chinensis (Pilg.) Rehd., Canarium pimela Koenig, Heteropanax fragrans (Roxb.) Seem. und Andrographis paniculata (Burm.f.) Nees verwendet, da sie die 5α-Reduktase (TypI und Ty-pII) hemmt und gleichzeitig eine Blockade von Androgen-Rezeptoren ermöglicht.
Die erfindungsgemäße Zusammensetzung umfasst eine Kombination wie in Anspruch 1 angegeben.

Im Allgemeinen kann der Extrakt aus der gesamten Pflanze oder einem Teil davon gewonnen werden, z.B. aus Blättern, Stengeln oder Ästen, der Rinde, Blüten, Früchten, Wurzeln oder dergleichen. Bevorzugt wird die Pflanzenquelle vor dem Extrahieren zerkleinert, zermalmt oder pulverisiert. Weitere optionale Verarbeitungsschritte sind Erhitzen, Halten unter Rückfluß, Filtrieren, Konzentrieren, Sprühtrocknen, Gefriertrocknen. Bevorzugt wird ein gezielter Isolierschritt angefügt, bei dem die extrahierte Probe aufgetrennt wird, z.B. über geeignete Chromatographie-Methoden, und die jeweiligen Fraktionen mit der gewünschten Wirkung isoliert und ggf. weiter aufgereinigt wird. So kann zum Beispiel die Zielisolierung durch Bestimmung und Bestätigung der jeweils gewünschten Aktivität, und/oder durch Prüfen auf einen substanziellen Gehalt von Flavonoiden und/oder bevorzugt von Phytosterolen, vor allem von beta-Sitosterol, Stigmasterol und Campesterol, erfolgen. Der Pflanzenextrakt der Komponente (ii) stellt besonders bevorzugt ein an Phytosterolen und/oder Flavonoiden reicher Extrakt dar, d.h. der Anteil an Phytosterolen und/oder Flavonoiden am gesamten Pflanzenextrakt der Komponente (ii) beträgt z.B. mindestens 50 Gew.-%, weiter bevorzugt mindestens 75 Gew.-% und insbesondere mindestens 90 Gew.-%.

Um vorrangig die bevorzugten steroidalen Wirkstoffe aus den genannten Pflanzen zu isolieren, werden sie bevorzugt mit organischen Lösungsmitteln extrahiert, beispielsweise mit Mehtanol, Ethanol, Hexanol, Glykol wie Ethylenglykol oder 1,3-Butylenglykol, Aceton, Hexan, Benzol, Toluol, Chloroform extrahiert. Ein besonders bevorzugtes Extraktionsmittel ist Ethanol.

Erfindungsgemäß ist es besonders bevorzugt, wenn entweder die Komponente (i) oder die Komponente (ii), besser noch gleichzeitig beide Komponenten die Typ1- oder die Typ2-5α-Reduktase und weiter bevorzugt beide Isoformen hemmt/hemmen, um einen multifunktionellen Wirkungsmechanismus zu ermöglichen, ohne weitere Wirkungssubstanzen beimischen zu müssen und dadurch möglicherweise störende Nebeneffekte zu erzeugen. So wird eine optimierte Wirkstoffkombination, die sich vor allem durch eine günstige Beeinflussung des positiven Haarwachstums beim Kopfhaar mit der Möglichkeit einer Re-Pigmentierung der Haare bei ansonsten starker Zurückdrängung des Haarwachstums an der Körperbehaarung (sowie beim so genannten "Damenbart") auszeichnet, dann erhalten, wenn als Wirkprinzip nur der Aromatase-Inhibitor -in Form eines Raps-Extrakts - mit dem Pflanzenextrakt der Komponente (ii) wie in Anspruch 1 festgelegt kombiniert wird, ohne weitere pharmakologische Wirksubstanzen beizufügen sondern nur noch, je nach gewünschter Formulierung, geeignete Trägerstoffe, Hilfsstoffe oder Additive. Dies ist besonders dann der Fall, wenn ein Aromatase-Inhibitor verwendet wird, der die Aromatase durch kovalente Bindung an die Aromatase hemmt, insbesondere beim Einsatz von 4-Hydroxy-Androsten-dion, 4- Acetoxy-Andostendion oder einem 4-Ester-Derivat davon, wobei die Estergruppe übliche Alkylgruppen wie Methyl-, Acetyl-, n- oder iso-Propyl-, n-, sec- oder t-Butylester enthalten kann.

Es wird angenommen, dass bei Verwendung der Komponente (i) gemäß Anspruch 1, in Kombination mit dem Pflanzenextrakt der Komponente (ii) gemäß Anspruch 1, ein multimodaler Wirkungsmechanismus ausgeübt wird, ohne daß es weiterer ggf. störender Wirkstoffzusätze bedarf, und zwar neben der Hemmung der Aromatase gleichzeitig eine Hemmung der 5α-Reduktase (TypI und TypII) und zusätzlich dazu eine Blockierung des Androgen-Rezeptors.

Die Mengen der oben genannten Wirksubstanz-Komponenten - d.h. jeweils Aromatese-Inhibitor, Sägepalmenextrakt und ggf. zusätzlicher 5α-Reduktase-Inhibitor - sind beispielsweise, jeweils unabhängig voneinander, Bereiche von 0,0001 bis 50 Gew.-%, vorzugsweise 0,001 bis 20 Gew.-%, weiter bevorzugt 0,01 bis 10 Gew.-% und insbesondere 0,1 bis 5 Gew.-%, jeweils bezogen auf die gesamte Zusammensetzung.

Zusätzlich zu den vorstehend genannten wirksamen Bestandteilen kann die erfindungsgemäße Zusammensetzung übliche Trägerstoffe, Hilfsstoffe oder Additive enthalten. Insbesondere kommen solche Zusatzstoffe in Betracht, die für topische Applikationsformen geeignet sind. Geeignete Träger- bzw. Zusatzstoffe schließen beispielsweise pflanzliche Öle wie Mandelöl, Olivenöl, Pfirsichkernöl, Erdnußöl, Ricinusöl u. dergl., Pflanzenextrakte, etherische Öle, Vitaminöle, Fette und fettähnliche Stoffe, Lipoide, Phosphatide, Kohlenwasserstoffe wie Paraffine, Vaseline, Lanolin, Wachse u. dergl., Detergention, weitere Hautwirkstoffe wie Lecithin, Wollfettalkohole, Carotin u. dergl., Hautnährstoffe, Parfums, kosmetische Stoffe, Alkohole, Wasser und Wassergemische, Glycerin, Glykole, Harnstoff, Talk, Konservierungsmittel, Sonnenschutzmittel, Farbstoffe wie Titanweiß und Zinkweiß, und Antioxidantien oder dergleichen sowie Mischungen aus den genannten Stoffen ein, ohne jedoch darauf beschränkt zu sein. Als Grundsubstanz dient im allgemeinen Wasser, so daß - üblicherweise unter Zusatz von Emulgatoren wie Fettalkoholsulfate, Alkaliseifen, Lecitine, Triethanolamin u. dergl. - eine O/W- oder W/O-Emulsion erhalten wird. Einsetzbar als Basismischung neben den Wirkungssubstanzen sind auch handelsübliche, herkömmliche Hautpflegemittel.
Geeignete Formulierungsformen für die erfindungsgemäße Zusammensetzung sind z.B. eine Salbe, eine Creme, ein Gel, eine Emulsion, eine Lotio, ein Spray, ein Puder, ein Öl oder dergleichen. Die erfindungsgemäße Zusammensetzung ist aber bevorzugt frei von bedenklich, unerwünschten oder sogar toxischen Zusätzen, insbesondere frei von metallischen Zusätzen wie Kupfer.

Die Erfindung wird nachfolgend anhand folgender Beispiele, die jedoch nicht einschränkend zu verstehen sind, näher erläutert. Angaben in % bedeuten Gewichts-% der jeweiligen Zusammensetzung.

### Beispiele 1-8:

In den nachfolgenden Beispielen 1-8 wurden die genannten Inhaltsstoffe (Wirkstoffe bzw. Wirkstoffgemische) zur Herstellung von Tinkturen zur Beeinflussung des Haarwachstums jeweils für Männer und Frauen eingesetzt.

### Haarwachstum Kopf Männer:

### Referenzbeispiel 1 (nicht Gegenstand der Ansprüche):

AQUA 14,24%, DIMETHYL ISOSORBIDE 10%, SERENOA SERRULATA FRUIT EXTRACT 0,99%, 4 - HYDROXY - ANDROSTENEDIONE 0,6%, URTICA DIOICA (NETTLE) EXTRACT 1%, TUSSILAGO FARFARA (COLTSFOOT) LEAF EXTRACT 0,2%, RETINYL PALMITATE 0,05%, TOCOPHEROL 0,02%, ZINC PCA 0,2%, EQUISETUM ARVENSE EXTRACT 0,2%, MALVA SYLVESTRIS (MALLOW) EXTRACT 0,4%, DISODIUM CYSTINYL DISUCCINATE 0,2%, PIROCTONE OLAMINE 0,2%, TRIDECYL SALICYLATE 0,2%, NIACINAMIDE 0,1%, HAMAMELIS VIRGINIANA (WITCH HAZEL) EXTRACT 0,4%, OLEA EUROPAEA (OLIVE) OIL UNSAPONIFIABLES 0,4%, BIOTIN 0,2%, UREA 0,25%, PEG-40 HYDROGENATED CASTOR OIL 0,05%, PROPYLENE GLYCOL 0,05%, LACTIC ACID 0,05%, und Rest ALCOHOL DENAT. (70%).

### Referenzbeispiel 2 (nicht Gegenstand der Ansprüche) :

AQUA 14,24%, DIMETHYL ISOSORBIDE 10%, SERENOA SERRULATA FRUIT EXTRACT 0,99%, ACETOXYANDROSTENEDIONE 0,6%, URTICA DIOICA (NETTLE) EXTRACT 1%, TUSSILAGO FARFARA (COLTSFOOT) LEAF EXTRACT 0,2%, RETINYL PALMITATE 0,05%, TOCOPHEROL 0,02%, ZINC PCA 0,2%, EQUISETUM ARVENSE EXTRACT 0,2%, MALVA SYLVESTRIS (MALLOW) EXTRACT 0,4%, DISODIUM CYSTINYL DISUCCINATE 0,2%, PIROCTONE OLAMINE 0,2%, TRIDECYL SALICYLATE 0,2%, NIACINAMIDE 0,1%, HAMAMELIS VIRGINIANA (WITCH HAZEL) EXTRACT 0,4%, OLEA EUROPAEA (OLIVE) OIL UNSAPONIFIABLES 0,4%, BIOTIN 0,2%, UREA 0,25%, PEG-40 HYDROGENATED CASTOR OIL 0,05%, PROPYLENE GLYCOL 0,05%, LACTIC ACID 0,05%, und Rest ALCOHOL DENAT. (70%).

### Referenzbeispiel 3 (nicht Gegenstand der Ansprüche):

AQUA 14,33%, DIMETHYL ISOSORBIDE 10%, SERENOA SERRULATA FRUIT EXTRACT 1%, GLYCINE SOJA (SOYBEAN) STEROLS 0,5%, URTICA DIOICA (NETTLE) EXTRACT 1%, TUSSILAGO FARFARA (COLTSFOOT) LEAF EXTRACT 0,2%, RETINYL PALMITATE 0,05%, TOCOPHEROL 0,02%, ZINC PCA 0,2%, EQUISETUM ARVENSE EXTRACT 0,2%, MALVA SYLVESTRIS (MALLOW) EXTRACT 0,4%, DISODIUM CYSTINYL DISUCCINATE 0,2%, PIROCTONE OLAMINE 0,2%, TRIDECYL SALICYLATE 0,2%, NIACINAMIDE 0,1%, HAMAMELIS VIRGINIANA (WITCH HAZEL) EXTRACT 0,4%, OLEA EUROPAEA (OLIVE) OIL UNSAPONIFIABLES 0,4%, BIOTIN 0,2%, UREA 0,25%, PEG-40 HYDROGENATED CASTOR OIL 0,05%, PROPYLENE GLYCOL 0,05%, LACTIC ACID 0,05%, und Rest ALCOHOL DENAT. (70%).

### Beispiel 4:

AQUA 14,33%, DIMETHYL ISOSORBIDE 10%, SERENOA SERRULATA FRUIT EXTRACT 1%, BRASSICA CAMPESTRIS (RAPESEED) STEROLS 0,5%, URTICA DIOICA (NETTLE) EXTRACT 1%, TUSSILAGO FARFARA (COLTSFOOT) LEAF EXTRACT 0,2%, RETINYL PALMITATE 0,05%, TOCOPHEROL 0,02%, ZINC PCA 0,2%, EQUISETUM ARVENSE EXTRACT 0,2%, MALVA SYLVESTRIS (MALLOW) EXTRACT 0,4%, DISODIUM CYSTINYL DISUCCINATE 0,2%, PIROCTONE OLAMINE 0,2%, TRIDECYL SALICYLATE 0,2%, NIACINAMIDE 0,1%, HAMAMELIS VIRGINIANA (WITCH HAZEL) EXTRACT 0,4%, OLEA EUROPAEA (OLIVE) OIL UNSAPONIFIABLES 0,4%, BIOTIN 0,2%, UREA 0,25%, PEG-40 HYDROGENATED CASTOR OIL 0,05%, PROPYLENE GLYCOL 0,05%, LACTIC ACID 0,05%, und Rest ALCOHOL DENAT. (70%).

### Haarwachstum Kopf Frauen:

### Referenzbeispiel 5 (nicht Gegenstand der Ansprüche):

AQUA 14,33%, DIMETHYL ISOSORBIDE 10%, 4 - HYDROXY - ANDROSTENEDIONE 0,7%, SERENOA SERRULATA FRUIT EXTRACT 0,8%, URTICA DIOICA (NETTLE) EXTRACT 1%, TUSSILAGO FARFARA (COLTSFOOT) LEAF EXTRACT 0,2%, RETINYL PALMITATE 0,05%, TOCOPHEROL 0,02%, ZINC PCA 0,2%, EQUISETUM ARVENSE EXTRACT 0,2%, MALVA SYLVESTRIS (MALLOW) EXTRACT 0,4%, DISODIUM CYSTINYL DISUCCINATE 0,2%, PIROCTONE OLAMINE 0,2%, TRIDECYL SALICYLATE 0,2%, NIACINAMIDE 0,1%, HAMAMELIS VIRGINIANA (WITCH HAZEL) EXTRACT 0,4%, OLEA EUROPAEA (OLIVE) OIL UNSAPONIFIABLES 0,4%, BIOTIN 0,2%, UREA 0,25%, PEG-40 HYDROGENATED CASTOR OIL 0,05%, PROPYLENE GLYCOL 0,05%, LACTIC ACID 0,05, und Rest ALCOHOL DENAT. (70%).

### Referenzbeispiel 6 (sicht Gegenstand der Ansprüche):

AQUA 14,33%, DIMETHYL ISOSORBIDE 19%, ACETOXYANDROSTENEDIONE 07%, SERENOA SERRULATA FRUIT EXTRACT 0,8%, URTICA DIOICA (NETTLE) EXTRACT 1%, TUSSILAGO FARFARA (COLTSFOOT) LEAF EXTRACT 0,2%, RETINYL PALMITATE 0,05%, TOCOPHEROL 0,02%, ZINC PCA 0,2%, EQUISETUM ARVENSE EXTRACT 0,2%, MALVA SYLVESTRIS (MALLOW) EXTRACT 0,4%, DISODIUM CYSTINYL DISUCCINATE 0,2%, PIROCTONE OLAMINE 0,2%, TRIDECYL SALICYLATE 0,2%, NIACINAMIDE 0,1%, HAMAMELIS VIRGINIANA (WITCH HAZEL) EXTRACT 0,4%, OLEA EUROPAEA (OLIVE) OIL UNSAPONIFIABLES 0,4%, BIOTIN 0,2%, UREA 0,25%, PEG-40 HYDROGENATED CASTOR OIL 0,05%, PROPYLENE GLYCOL 0,05%, LACTIC ACID 0,05%, und Rest ALCOHOL DENAT. (70%).

### Referenzbeispiel 7 (nicht Gegenstand der Ansprüche):

AQUA 14,23%, DIMETHYL ISOSORBIDE 10%, GLYCINE SOJA (SOYBEAN) STEROLS 1%, SERENOA SERRULATA FRUIT EXTRACT 0,6%, URTICA DIOICA (NETTLE) EXTRACT 1%, TUSSILAGO FARFARA (COLTSFOOT) LEAF EXTRACT 0,2%, RETINYL PALMITATE 0,05%, TOCOPHEROL 0,02%, ZINC PCA 0,2%, EQUISETUM ARVENSE EXTRACT 0,2%, MALVA SYLVESTRIS (MALLOW) EXTRACT 0,4%, DISODIUM CYSTINYL DISUCCINATE 0,2%, PIROCTONE OLAMINE 0,2%, TRIDECYL SALICYLATE 0,2%, NIACINAMIDE 0,1%, HAMAMELIS VIRGINIANA (WITCH HAZEL) EXTRACT 0,4%, OLEA EUROPAEA (OLIVE) OIL UNSAPONIFIABLES 0,4%, BIOTIN 0,2%, UREA 0,25%, PEG-40 HYDROGENATED CASTOR OIL 0,05%, PROPYLENE GLYCOL 0,05%, LACTIC ACID 0,05%, und Rest ALCOHOL DENAT. (70%).

### Beispiel 8:

AQUA 14,23%, DIMETHYL ISOSORBIDE 10%, BRASSICA CAMPESTRIS (RAPESEED) STEROLS 1%, SERENOA SERRULATA FRUIT EXTRACT 0,6%, URTICA DIOICA (NETTLE) EXTRACT 1%, TUSSILAGO FARFARA (COLTSFOOT) LEAF EXTRACT 0,2%, RETINYL PALMITATE 0,05%, TOCOPHEROL 0,02%, ZINC PCA 0,2%, EQUISETUM ARVENSE EXTRACT 0,2%, MALVA SYLVESTRIS (MALLOW) EXTRACT 0,4%, DISODIUM CYSTINYL DISUCCINATE 0,2%, PIROCTONE OLAMINE 0,2%, TRIDECYL SALICYLATE 0,2%, NIACINAMIDE 0,1%, HAMAMELIS VIRGINIANA (WITCH HAZEL) EXTRACT 0,4%, OLEA EUROPAEA (OLIVE) OIL UNSAPONIFIABLES 0,4%, BIOTIN 0,2%, UREA 0,25%, PEG-40 HYDROGENATED CASTOR OIL 0,05%, PROPYLENE GLYCOL 0,05%, LACTIC ACID 0,05%, und Rest ALCOHOL DENAT. (70%).

### Beispiele 9-14:

In den nachfolgenden Beispiele 9-14 wurden die genannten Inhaltsstoffe zur Herstellung von Cremes zur Beeinflussung des Haar - Neuwachstums und der Haarentfernung (Gesicht und Körper) eingesetzt.

### Haarentfernung Körper :

### Beispiel 9:

DIMETHYL ISOSORBIDE 6%, PERSEA GRATISSIMA (AVOCADO) OIL 4%, OCTYLDODECANOL 3,9%, TRIDECYL SALICYLATE 2%, C12-13 ALKYL LACTA-TE 2%, CETEARYL ISONONANOATE 2%, CAPRYLIC/CAPRIC TRIGLYCERIDE 1,9476%, POLYGLYCERYL-3 METHYLGLUCOSE DISTEARATE 1,9%, AMMONIUM ACRYLOYLDIMETHYLTAURATE/VP COPOLYMER 0,85%, SERENOA SERRULATA FRUIT EXTRACT 0,9%, BRASSICA CAMPESTRIS (RAPESEED) STEROLS 0,5%, GLYCINE SOJA (SOYBEAN) STEROLS 0,5%, TOCOPHERYL ACETATE 0,9%, TOCOPHEROL 0,0175%, CAPRYLOYL GLYCINE 0,1%, TRIBEHENIN 0,9%, GLYCERIN 0,9552%, XYLITOL 0,9%, SORBITOL, 0,9%, PALMATINE 0,0024%, COCO-GLUCOSIDE 0,052%, ETHYLHEXYLGLYCERIN 0,3%, LACTIC ACID 0,12%, SORBITAN LAURATE 0,09%, POLOXAMER 407 0,5%, NYLON-12 0,06%, LECITHIN 0,028%, ASCORBYL PALMITATE 0,0175%, AMMONIA 0,0175, PHENOXYETHANOL 0,8%, XANTHAN GUM 0,1%, und Rest AQUA (66,7423%).

### Referenzbeispiel 10 (nicht Gegenstand der Ansprüche):

DIMETHYL ISOSORBIDE 6%, PERSEA GRATISSIMA (AVOCADO) OIL 4%, OCTYLDODECANOL 3,9%, TRIDECYL SALICYLATE 2%, C12-13 ALKYL LACTA-TE 2%, CETEARYL ISONONANOATE 2%, CAPRYLIC/CAPRIC TRIGLYCERIDE 1,9476%, POLYGLYCERYL-3 METHYLGLUCOSE DISTEARATE 1,9%, AMMONIUM ACRYLOYLDIMETHYLTAURATE/VP COPOLYMER 0,85%, SERENOA SERRULATA FRUIT EXTRACT 0,9%, ACETOXYANDROSTENEDIONE 0,5%, TOCOPHERYL ACETATE 0,9%, TOCOPHEROL 0,0175%, CAPRYLOYL GLYCINE 0,1%, TRIBEHENIN 0,9%, GLYCERIN 0,9552%, XYLITOL 0,9%, SORBITOL 0,9%, PALMATINE 0,024%, COCO-GLUCOSIDE 0,052%, ETHYLHEXYLGLYCERIN 0,3%, LACTIC ACID 0,12%, SORBITAN LAURATE 0,09%, POLOXAMER 407 0,5%, NYLON-12 0,06%, LECITHIN 0,028%, ASCORBYL PALMITATE 0,0175%, AMMONIA 0,0175%, PHENOXYETHANOL 0,8%, XANTHANGUM 0,1%, und Rest AQUA (67,2423%).

### Referenzbeispiel 11 (nicht Gegenstand der Ansprüche):

DIMETHYL ISOSORBIDE 6%, PERSEA GRATISSIMA (AVOCADO) OIL 4%, OCTYLDODECANOL 3,9%, TRIDECYL SALICYLATE 2%, C12-13 ALKYL LACTA-TE 2%, CETEARYL ISONONANOATE 2%, CAPRYLIC/CAPRIC TRIGLYCERIDE 1,9476%, POLYGLYCERYL-3 METHYLGLUCOSE DISTEARATE 1,9%, AMMONIUM ACRYLOYLDIMETHYLTAURATE/VP COPOLYMER 0,85%, SERENOA SERRULATA FRUIT EXTRACT 0,9%, 4 - HYDROXY - ANDROSTENEDIONE 0,5%, TOCOPHERYL ACETATE 0,9%, TOCOPHEROL 0,0175%, CAPRYLOYL GLYCINE 0,1%, TRIBEHENIN 0,9%, GLYCERIN 0,9552, XYLITOL 0,9%, SORBITOL 0,9%, PALMATINE 0,024%, COCO-GLUCOSIDE 0,052%, ETHYLHEXYLGLYCERIN 0,3%, LACTIC ACID 0,12%, SORBITAN LAURATE 0,09%, POLOXAMER 407 0,5%, NYLON-12 0,06%, LECITHIN 0,028%, ASCORBYL PALMITATE 0,0175%, AMMONIA 0,0175, PHENOXYETHANOL 0,8%, XANTHAN GUM 0,1%, und Rest AQUA (67,2423%).

### Haarentfernung Gesicht:

### Beispiel 12:

DIMETHYL ISOSORBIDE 6%, PERSEA GRATISSIMA (AVOCADO) OIL 4,5%, OCTYLDODECANOL 4,4%, TRIDECYL SALICYLATE 2%, C12-13 ALKYL LACTA-TE 2%, CETEARYL ISONONANOATE 2%, CAPRYLIC/CAPRIC TRIGLYCERIDE 1,9476%, POLYGLYCERYL-3 METHYLGLUCOSE DISTEARATE 1,9%, BRASSICA CAMPESTRIS (RAPESEED) STEROLS 0,5%, GLYCINE SOJA (SOYBEAN) STEROLS 0,48%, SERENOA SERRULATA FRUIT EXTRACT 0,9%, CAPRYLOYL GLYCINE 0,1%, TRIBEHENIN 0,9%, GLYCERIN 0,9552%, XYLITOL 0,9%, SORBITOL 0,9%, AMMONIUM ACRYLOYLDIMETHYLTAURATE/VP COPOLYMER 1,25%, PALMATINE 0,024%, COCO-GLUCOSIDE 0,052%, TOCOPHERYL ACETATE 0,9%, TOCOPHEROL 0,0175%, ETHYLHEXYLGLYCERIN 0,3%, LACTIC ACID 0,12%, SORBITAN LAURATE 0,09%, POLOXAMER 407 0,5%, NYLON-12 0,06%, LECITHIN 0,028%, ASCORBYL PALMITATE 0,0175%, AMMONIA 0,0175%, PHENOXYETHANOL 0,8%, XANTHAN GUM 0,1%, und Rest AQUA (65,3407%).

### Referenzbeispiel 13 (nicht Gegenstand der Ansprüche):

DIMETHYL ISOSORBIDE 6%, PERSEA GRATISSIMA (AVOCADO) OIL 4,5%, OCTYLDODECANOL 4,4%, TRIDECYL SALICYLATE 2%, C12-13 ALKYL LACTA-TE 2%, CETEARYL ISONONANOATE 2%, CAPRYLIC/CAPRIC TRIGLYCERIDE 1,9476%, POLYGLYCERYL-3 METHYLGLUCOSE DISTEARATE 1,9%, ACETOXYANDROSTENEDIONE 0,68, SERENOA SERRULATA FRUIT EXTRACT 0,9%, CAPRYLOYL GLYCINE 0,1%, TRIBEHENIN 0,9%, GLYCERIN 0,9552%, XYLITOL 0,9%, SORBITOL 0,9%, AMMONIUM ACRYLOYLDIMETHYL-TAURATE/VP COPOLYMER 1,25%, PALMATINE 0,024%, COCO-GLUCOSIDE 0,52%, TOCOPHERYL ACETATE 0,9%, TOCOPHEROL 0,0175%, ETHYLHEXYLGLYCERIN 0,3%, LACTIC ACID 0,12%, SORBITAN LAURATE 0,09%, POLOXAMER 407 0,5%, NYLON-12 0,06%, LECITHIN 0,028%, ASCORBYL PALMITATE 0,0175%, AMMONIA 0,0175%, PHENOXYETHANOL 0,8%, XANTHAN GUM 0,1%, und Rest AQUA (65,6407%).

### Referenzbeispiel 14 (nicht Gegenstand der Ansprüche):

DIMETHYL ISOSORBIDE 6%, PERSEA GRATISSIMA (AVOCADO) OIL 4,5%, OCTYLDODECANOL 4,4%, TRIDECYL SALICYLATE 2%, C12-13 ALKYL LACTA-TE 2%, CETEARYL ISONONANOATE 2%, CAPRYLIC/CAPRIC TRIGLYCERIDE 1,9476%, POLYGLYCERYL-3 METHYLGLUCOSE DISTEARATE 1,9%, 4 - HYDROXY - ANDROSTENEDIONE 0,68%, SERENOA SERRULATA FRUIT EXTRACT 0,9%, CAPRYLOYL GLYCINE 0,1%, TRIBEHENIN 0,9%, GLYCERIN 0,9552%, XYLITOL 0,9%, SORBITOL 0,9%, AMMONIUM ACRYLOYLDIMETHYLTAURATE/VP COPOLYMER 1,25%, PALMATINE 0,024%, COCO-GLUCOSIDE 0,052%, TOCOPHERYL ACETATE 0,9%, TOCOPHEROL 0,0175%, ETHYLHEXYLGLYCERIN 0,3%, LACTIC ACID 0,12%, SORBITAN LAURATE 0,09%, POLOXAMER 407 0,5%, NYLON-12 0,06%, LECITHIN 0,028%, ASCORBYL PALMITATE 0,0175%, AMMONIA 0,0175%, PHENOXYETHANOL 0,8%, XANTHAN GUM 0,1%, und Rest AQUA (65,6407%).

Die nachfolgenden Anwendungs-Beispiele 1 und 2 geben die Ergebnisse mit repräsentativ ausgewählten Zusammensetzungen der Zubereitungsbeispiele (1-14) bei Fällen mit Problemen des Haarwachstums bzw. bei überschüssiger Behaarung an Körper und Gesicht wieder.

### Anwendungsbeispiele 1 und 2:

### Anwendungsbeispiel 1: Einfluss auf die Kopfbehaarung bei Frauen und Männern mit genetischer Alopecie:

Bei 10 Frauen im Alter zwischen 40 und 60 Jahren und bei 12 Männern im Alter zwischen 34 und 64 Jahren mit starker genetischer Alopecie wurden Lotio - Zubereitungen mit folgenden Wirkstoffkombinationen angewendet:
Bei den Frauen: eine Lotion, mit der Zusammensetzung nach Referenzbeispiel 6 (n=4) bzw. nach Referenzbeispiel 5 (n=6)
Bei den Männern: eine Lotion, mit der Zusammensetzung nach Referenzbeispiel 2 (n=12).

### Ergebnisse:

Bei allen Männern und Frauen zeigte sich bereits nach 3 Monaten sowohl eine positive Beeinflussung des Haarwachstums (Haarmenge) als auch eine Zunahme der Dicke der Haare. Nach 6 Monaten zeigte sich neben einer äußerst bemerkenswerten Zunahme des Haarwachstums (Haarmenge) eine erhebliche Zunahme der Haardicke. Überraschender Weise zeigte sich bei fast allen Frauen (n = 9) und bei fast allen Männern (n = 10), eine Re - Pigmentierung der Haare und damit eine Rückkehr zur ursprünglichen Haarfarbe.

### Anwendungsbeispiel 2: überschüssige Beinbehaarung (n=35) und sogenannter Damenbart (n=6) bei Frauen:

### Anwendungen auf die Beinbehaarung bei Frauen :

Frauen mit starker Beinbehaarung (n = 35) bekamen über 6 Monate eine Creme mit der Zusammensetzung nach Referenzbeispiel 10 (N=15) oder Referenzbeispiel 11 (n=20).

### Ergebnisse:

Die Untersuchung erstreckte sich über 180 Tage mit Untersuchungszeitpunkten im Abstand von 30 Tagen. Die Ergebnisse waren überraschend. Bereits nach 30 Tagen zeigte sich eine Abnahme der Haarmenge und Haardicke. Nach 180 Tagen reduzierte sich die Anzahl der Haare pro Untersuchungsareal um 89%, während bei beinahe bei 70% der verbliebenen Haare eine vollständige DePigmentierung und bei mehr 95% der Frauen die verbliebenen Haare erheblich an Dicke abnahmen, also ganz fein wurden. Die Ergebnisse für beide Cremezubereitungen waren identisch, zeigten also beide die gleiche biologische Effektivität.

Gleiche Effekte zeigten sich bei 6 Frauen mit so genanntem Damenbart bei der Anwendung einer Creme der Zusammensetzung wie im Referenzbeispiel 13 dargestellt:
Es wird postuliert, daß die gewählten Wirkstoffkombinationen dazu führen, dass sowohl die lokale Östradiol- als auch die lokale Dihydrotestosteron-Bildung unterdrückt werden, und daß darüber hinaus über die Blockierung der Androgen-Rezeptoren aufgrund zum Beispiel der Wirkung des Sägepalmen-Extrakts auch die mögliche Wirkung des zirkulierenden Dihydrotestosterons in der Haar Papille blockiert wird.

## Patentansprüche

1. Zusammensetzung, insbesondere mit der Eigenschaft einer Beeinflussung des Haarwachstums, **dadurch gekennzeichnet, daß** die Zusammensetzung eine Kombination von
(i) mindestens einem Aromatase-Inhibitor, der aus der Gruppe von Aromatase-Inhibition aufweisenden Extrakten von Raps ausgewählt ist, und
(ii) mindestens einem Pflanzenextrakt, der eine oder mehrere aus der Pflanze extrahierte Wirksubstanz(en) enthält, die aus der aus 5α-Reduktase Typ I- und/oder Typ II-Inhibitoren und Androgenrezeptor-Blockern bestehenden Gruppe ausgewählt ist (sind), wobei der mindestens eine Pflanzenextrakt ein Extrakt aus Sägepalme (Serenoa Serrulata Fruit Extract) ist, umfasst.

2. Zusammensetzung gemäß Anspruch 1, **dadurch gekennzeichnet, daß** der Aromatase-Inhibitor die Eigenschaft hat, auch die 5α-Reduktase zu hemmen.

3. Zusammensetzung gemäß Anspruch 1, **dadurch gekennzeichnet, daß** die Komponente (i) allein ein Extrakt aus Raps ist.

4. Zusammensetzung gemäß Anspruch 1, **dadurch gekennzeichnet, daß** die Komponente (i) derart ist, daß der Extrakt aus Raps mit einem chemisch-synthetischen Aromatase-Inhibitor kombiniert ist.

5. Zusammensetzung gemäß einem der vorangehenden Ansprüche, **dadurch gekennzeichnet, daß** der Sägepalmen-Extrakt ein Ethanolextrakt von Sägepalmenfrüchten ist.

6. Zusammensetzung gemäß einem der vorangehenden Ansprüche, **dadurch gekennzeichnet, dass** der Pflanzenextrakt der Komponente (ii) einen an Phytosterolen und/oder Flavonoiden reichen Extrakt darstellt.

7. Zusammensetzung gemäß einem der Ansprüche 4-6, **dadurch gekennzeichnet, daß** der chemisch-synthetische Aromatase-Inhibitor 4-Hydroxy-androsten-dion oder ein 4-Carbonsäureester-Derivat davon ist.

8. Zusammensetzung gemäß Anspruch 7, **dadurch gekennzeichnet, daß** das 4-Carbonsäureester-Derivat 4-Acetoxy-androstendion ist.

9. Zusammensetzung gemäß einem der vorangehenden Ansprüche, **dadurch gekennzeichnet, daß** neben den Komponenten (i) und (ii) keine weiteren Wirksubstanzen beigefügt sind, sondern allenfalls geeignete Trägerstoffe, Hilfsstoffe oder Additive, insbesondere keine toxische Metalle wie Kupfer beigefügt sind.

10. Zusammensetzung gemäß einem der vorangehenden Ansprüche, **dadurch gekennzeichnet, daß** sie zur topischen Applikation geeignet formuliert ist.

11. Verwendung einer Zusammensetzung gemäß einem der Ansprüche 1-10, für kosmetische Anwendungen.

12. Nicht-therapeutische Verwendung einer Zusammensetzung gemäß einem der Ansprüche 1 bis 10,
• zur Verringerung des Haarwachstums am Körper oder im Gesicht,
• zur Haarentfernung,
• zur Depigmentierung der Körperbehaarung der Frau, und/oder
• zur Förderung des Haarwachstums an der Skalp-Kopfbehaarung, wobei eine Pigmentierung von Haaren erhalten bleibt oder eine Re-Pigmentierung von Haaren gefördert wird.

## Claims

1. Composition, in particular with the property of influencing hair growth, **characterized in that** the composition comprises a combination of
(i) at least one aromatase-inhibitor, selected from the group of aromatase-inhibition exhibiting extracts of rapeseed, and
(ii) at least one plant extract that contains one or more active ingredient substance(s) extracted from the plant, which is (are) selected from the group consisting of 5α-reductase type I- and/or type II-inhibitors and androgen receptor-blockers, wherein the at least one plant extract is an extract of saw palmetto (Serenoa Serrulata Fruit Extract).

2. Composition according to claim 1, **characterized in that** the aromatase-inhibitor has the property of additionally inhibiting the 5α-reductase.

3. Composition according to claim 1, **characterized in that** the component (i) is solely an extract of rapeseed,

4. Composition according to claim 1, **characterized in that** the component (i) is such that the extract of rapeseed is combined with a chemical-synthetic aromatase inhibitor.

5. Composition according to any of the preceding claims, **characterized in that** the saw palmetto extract is an ethanol extract of saw palmetto fruits.

6. Composition according to any of the preceding claims, **characterized in that** the plant extract of component (ii) is an extract rich in phytosterols and/or flavonoids.

7. Composition according to any of claims 4 - 6, **characterized in that** the chemical-synthetic aromatase inhibitor is 4-hydroxy-androstene-dione or a 4-carboxylic acid ester-derivative thereof.

8. Composition according to claim 7, **characterized in that** the 4-carboxylic acid ester-derivative is 4-acetoxy-androstene-dione.

9. Composition according to any of the preceding claims, **characterized in that** besides the component (i) and (ii), no further active substances are added, in particular no toxic metals such as copper are added, but, at the most, suitable carriers, excipients, or additives.

10. Composition according to any of the preceding claims, **characterized in that** it is formulated suitable for topical application.

11. Use of a composition according to any of claims 1 - 10, for cosmetic use.

12. Non-therapeutic use of a composition according to any of claims 1 - 10,
- for reducing the hair growth of the body hair or facial hair,
- for removal of hair,
- for depigmentation on the body hair in woman, and/or
- for promoting hair growth on the scalp head hair, wherein a pigmentation of hair is maintained or a re-pigmentation of hair is promoted.

## Revendications

1. Composition, ayant en particulier la propriété d'influencer la croissance pileuse ou capillaire, **caractérisée en ce que** la composition comprend une combinaison de
(i) au moins un inhibiteur d'aromatase, qui est choisi dans le groupe des extraits de colza présentant une inhibition de l'aromatase, et
(ii) au moins un extrait végétal, qui contient une ou plusieurs substances actives extraites de la plante, qui est choisie dans le groupe constitué par les inhibiteurs de 5α-réductase de type I et/ou de type II et les bloquants du récepteur de l'androgène, le au moins un extrait végétal étant un extrait de chou palmiste (Serenoa Serrulata Fruit Extract).

2. Composition selon la revendication 1, **caractérisée en ce que** l'inhibiteur d'aromatase a la propriété d'inhiber également la 5α-réductase.

3. Composition selon la revendication 1, **caractérisée en ce que** le composant (i) est seulement un extrait de colza.

4. Composition selon la revendication 1, **caractérisée en ce que** le composant (i) est de telle sorte que l'extrait de colza est combiné à un inhibiteur d'aromatase chimico-synthétique.

5. Composition selon l'une des revendications précédentes, **caractérisée en ce que** l'extrait de chou palmiste est un extrait éthanolique des fruits du chou palmiste.

6. Composition selon l'une des revendications précédentes, **caractérisée en ce que** l'extrait végétal du composant (ii) forme un extrait riche en phytostérols et/ou en flavonoïdes.

7. Composition selon l'une des revendications 4 à 6, **caractérisée en ce que** l'inhibiteur d'aromatase chimico-synthétique est la 4-hydroxy-androstène-dione ou un dérivé d'ester d'acide 4-carboxylique de celle-ci.

8. Composition selon la revendication 7, **caractérisée en ce que** le dérivé d'ester d'acide 4-carboxylique est la 4-acétoxyandrostène-dione.

9. Composition selon l'une des revendications précédentes, **caractérisée en ce qu'**aucune autre substance active n'est ajoutée hormis les composants (i) et (ii), sauf à la rigueur des substances supports, auxiliaires ou des additifs appropriés, en particulier aucun métal toxique comme le cuivre n'est ajouté.

10. Composition selon l'une des revendications précédentes, **caractérisée en ce qu'**elle est formulée pour être appropriée à une application topique.

11. Utilisation d'une composition selon l'une des revendications 1 à 10, pour des applications cosmétiques.

12. Utilisation non thérapeutique d'une composition selon l'une des revendications 1 à 10,
• pour la réduction de la croissance pileuse sur le corps ou sur le visage,
• pour l'élimination des cheveux/poils,
• pour la dépigmentation des poils chez la femme, et/ou
• pour favoriser la croissance capillaire sur le cuir chevelu, une pigmentation des cheveux étant conservée ou une re-pigmentation des cheveux étant favorisée.
